# EUROPEAN PATENT SPECIFICATION

(11) **EP 0 837 656 B1**
(45) Date of publication and mention of the grant of the patent: **03.12.2003**
(21) Application number: 96924414.4
(22) Date of filing: 10.07.1996
(51) Int. Cl.: A61B 17/70

(54) **A POLYAXIAL LOCKING MECHANISM**
MEHRACHSIGER VERRIEGELUNGSMECHANISMUS
MECANISME DE VERROUILLAGE PIVOTANT SUR PLUSIEURS AXES

(30) Priority: 13.07.1995 US 502285; 14.07.1995 US 502809; 14.07.1995 US 502803; 13.10.1995 US 542540; 13.10.1995 US 542539; 13.10.1995 US 542542; 13.10.1995 US 542527
(43) Date of publication of application: 29.04.1998
(73) Proprietor: Fastenetix, L.L.C., Summit, NJ 07901 (US)
(72) Inventor: ERRICO, Joseph, P., Rockville, MD 20850 (US); ERRICO, Thomas, J., Summit, NJ 07901 (US); RALPH, James, D., Oakland, NJ 07436 (US)
(74) Representative: Gates, Marie Christina Esther
(86) International application number: US9611503
(87) International publication number: WO97002786

(56) References cited:
- EP-A- 0 778 007
- WO-A-88/03781
- WO-A-94/00066
- FR-A- 2 682 280
- FR-A- 2 704 133
- US-A- 5 352 226
- US-A- 5 443 467
- US-A- 5 476 464
- US-A- 5 534 001

## Description

### BACKGROUND OF THE INVENTION

### 1. Field of the Invention

This invention relates generally to a mechanism for polyaxially coupling and locking orthopaedic apparatus together so as to provide maximum surgical freedom and ease of use.

### 2. Description of the Prior Art

A variety of orthopaedic implant devices have been disclosed in the art for providing support to healing and/or fusing bone segments. These devices include bone plates, artificial joints, and rod immobilization implants. While affixation of such devices in many areas of the human body is often technically difficult, the need for variable angulability in implant devices which are used to immobilized segments of the spinal column is especially desirable. The spine is a highly complex system of bones and connective tissues which houses and protects critical elements of the nervous system and the arterial and veinous bodies in close proximity thereto. A variety of systems have been disclosed in the art which achieve this immobilization by implanting artificial assemblies in or on the spinal column.

These assemblies may be classified as anterior, posterior, or lateral implants. As the classification suggests, lateral and anterior assemblies are coupled to the anterior portion of the spine, which is the sequence of vertebral bodies. Posterior implants are attached to the back of the spinal column, generally hooking under the lamina and entering into the central canal, attaching to the transverse process, or coupling through the pedicle bone. The present invention relates to all such spinal fixation devices for immobilizing and altering the alignment of the spine by means of affixing at least one elongate rod to the sequence of selected bones.

These "rod assemblies" have a variety of pieces, including hooks, pedicle screws, and sacral blocks, each of which comprise a plurality of screws which are coupled to the rod. Pedicle screws are implanted through the posterior lateral surfaces of the laminae, through the pedicles, and into their respective vertebral bodies. The hooks are inserted under the lamina. The sacral block is coupled to the sacrum and receives the extreme end of the rod.It is the aligning influence of the rod forces the spine to which it is affixed, to conform to a more proper shape.

It has been identified, however, that a considerable difficulty may be associated with inserting screws, hooks, and sacral blocks along a misaligned curvature and simultaneously exactly positioning the rod relative thereto such that the receiving portions of sequential elements are aligned so that the rod can be passed therethrough without substantial advance contouring of the rod. Attempts at achieving proper alignment with fixed headed screws, hooks, and sacral blocks is understood to require considerably longer operating time, which is known to increase the incidence of complications associated with surgery. Often such alignments, with such fixed axes devices could not be achieved, and the entire instrumentationing effort would end unsuccessfully.

The art contains a variety of attempts at providing instrumentation which permit a freedom with respect to angulation of the screw and the coupling element. These teachings, however, have generally been complex, and inadequately reliable with respect to durability. The considerable drawbacks associated with the prior art systems include complexity, difficulty properly positioning the rod and coupling elements, and the tedious manipulation of the many parts associated with the complex devices.

EP 0 778 007 A describes a locking mechanism for use with orthopaedic implantation devices which comprises a body, a ring and a nut; the body including a threaded upper portion, two legs projecting from its lower end, and a channel being defined between the two legs. The channel accommodates one of the elongate components to be assembled, the legs being capable of moving apart or closer together; at least one bearing surface formed on the lateral face of the body or of the legs, forming an axial stop, and a transverse aperture in which the second elongate component to be assembled may be engaged; the ring can be engaged over the body until it comes to bear on the stop, and is dimensioned in such a way that in this position it lies around the legs; the nut can be screwed onto the threaded upper portion of the body.

One device known from the related art is French Patent No. 2,682,280, which teaches a fixation head of tuning fork shape that includes a means for solidly attaching a spinal osetosynthesis rod. The tuning fork shape defines a hollow housing open on three sides and bounded by a bottom and two side branches and comprises a bead capable of being positioned in the bottom of the housing between the two branches. The bead includes a slot capable of receiving at its bottom the rod and means for solidly attaching the rod and the bead with the head. The means consist in the fact that at least one of the two branches exhibits elastic deformation and that they comprise means for returning the two branches together towards one another. Nevertheless, the fixation system taught in French Patent No. 2,682,280 remains constrained to the geometry and physical properties of the fixed head screw upon which it is based. Accordingly, it likely shares the general aforementioned difficulties associated with screw insertion and spinal fixation system alignment. Additionally, the fixation system taught in French Patent No. 2,682,280 allows the possibility that forces acting on the rod may dislodge it from the fixation head, because the resistance offered by the at least one deformable branch may be readily over come. Thus, despite developments in the related art considerable drawbacks associated with the prior art systems remain."

It is, therefore, the principal object of the present invention to provide a coupling mechanism, which may be incorporated into a variety of different orthopaedic devices which provides a polyaxial freedom of implantation angulation between two elements, i.e., a rod and screw.

In addition, it is an object of the present invention to provide such an assembly which comprises a reduced number of elements, and which correspondingly provides for expeditious implantation.

Accordingly it is also an object of the present invention to provide an assembly which is reliable, durable, and provides long term fixation support.

Other objects of the present invention not explicitly stated will be set forth and will be more clearly understood in conjunction with the descriptions of the preferred embodiments disclosed hereafter.

### SUMMARY OF THE INVENTION

The preceding objects of the invention are achieved by the present invention which is a mechanism for flexibly coupling orthopaedic implant elements together and locking them together. The mechanism itself, which will be described more fully hereinbelow in pedicle screw, lamina hooks, and sacral block embodiments, generally comprise three primary elements. The first element has a curvate head portion; the second element has a portion thereof having a colletted and tapered exterior and an curvate interior volume for receiving the curvate head of the first element. A tapered locking collar is positioned around the tapered colletted portion of the second element such that selected translation of the collar relative to the second element causes the colletted portion to contract and the interior volume to crush lock to the curvate head.

More particularly, with respect to the pedicle screw embodiment of this invention, the polyaxial screw and coupling element assembly of the present invention comprises a bone screw having a head which is curvate in shape, for example semi-spherical, and a coupling element mounted thereon so as to be free to rotate prior to the secure fixation of the rod thereto, and which may be securely locked in a given angulation once the rod is received by the coupling element. The coupling element has a generally cylindrical main body portion, a locking collar, a removable external rod securing sleeve, and a top locking nut.

The coupling element may be conceptually divided into a lower socket portion, and a rod and nut receiving portion. In a first embodiment, in which the rod is received into the side of the coupling element, the rod and nut receiving portion may be subdivided into the intermediate rod receiving portion and the top nut receiving portion. (In the alternative embodiment in which the coupling element receives the rod from the top, the subdivision is unnecessary.)

The lower socket portion includes an interior chamber having an opening at the bottom thereof. The interior chamber is ideally suited for receiving therein the head of the screw such that the screw and the coupling element are held together in a rotationally and angularly free relationship. The external surface of the socket portion includes at least one vertical slot which is provided so that the opening in the bottom of the element may expand to receive the head of the screw, which has a major diameter which is larger than the unexpanded opening, such that the head of the screw may enter into the interior chamber. The at least one slot resiliently expands to permit the head of the screw to enter, and subsequently contracts into its original position once the head is fully inserted, therein inhibiting the screw head from being retracted. The head of the screw and the interior chamber are, however, free to rotate and angulate relative to one another.

The exterior of the lower socket portion of the coupling element, into which the screw head is inserted, tapers outward slightly toward the bottom of the element, therein having a slightly wider diameter at the bottom than at the top thereof. A locking collar, having a diameter equal to, or slightly larger than the top of the lower portion, but less than the diameter of the bottom of the lower portion, is initially disposed about the coupling element with the bottom of the locking collar resting against the widening surface of the element. The top of the collar may include two opposing grooves, or notches, onto which the rod is initially placed. Displacement of the locking collar downward causes the at least one vertical slot in the lower socket portion of the coupling element to narrow, therein causing the inner surface of the interior chamber to move radially inward, contacting the head of the screw, and locking thereto, thereby inhibiting further swingability.

The intermediate portion of the coupling element comprises a side receiving channel wherein the rod of the implant apparatus is mounted. More particularly, at a position above the lower portion, a channel is formed in the side of the generally cylindrical body, therein providing a receiving locus into which a support rod may nest. In order that the rod may be securely held within the receiving locus, an external rod securing sleeve is provided. The external rod securing sleeve is generally cylindrical in shape, having a hollow center for sliding over the top of the coupling element. The bottom of the cylindrical sleeve may include opposing grooves, similar to the grooves in the top of the locking collar. The grooves are positioned and designed to mate with the top of the rod, and to lock thereto upon the application of a downward force. The grooves of the sleeve, however, are preferably deeper than those of the locking collar, enabling the sleeve to encompass a larger angular section of the rod, thereby securely locking the rod in the rod receiving locus between the grooves of the sleeve and the grooves of the locking collar. In addition, the receiving locus is necessarily wider than the rod which is to be placed therein. This dimension relationship is required so that the sleeve may be forced down onto the rod, and that the rod may in turn force the locking collar downward. The rod, therefore, must be able to translate downward relative to the coupling element, within the receiving locus.

The upper portion of the coupling element comprises a threading onto which a locking nut may be inserted, therein providing a downward force onto the rod securing sleeve. The downward force of the sleeve is translated to a downward force of the rod, and on the locking collar. The locking collar is forced downward by the rod, and locks the screw in the interior chamber of the coupling element.

Each portion of the coupling element (lower, intermediate, and upper) includes a central bore, aligned with one another, and which extends axially from the top of the coupling element into the interior chamber. The screw head correspondingly includes a recess, which is alignable with the central bore of the coupling element, whereby a screw-driving instrument may be inserted through the central bore, into the recess in the screw, and utilized to drive the screw into the bone.

The first step in the process of implanting this embodiment of the invention is to insert the head of the screw into the interior chamber of the coupling element. Once it has been inserted, the angle of insertion at which the screw will have the greatest holding strength relative to the loading which the rod system will be applying thereto must be determined. Once this angle has been found, the screw and the coupling element are aligned with respect to one another so that a screw-driving tool may be inserted down the central bore of the coupling element, into the recess in the head of the screw, and thereby be rotationally inserted into the bone. Subsequent to the insertion of the screw, the screw-driving device is removed from the assembly, therein permitting the coupling element to rotate and change angular alignment relative to the screw.

In this position, the locking collar of the coupling element has not yet been forced downward to lock the screw to the coupling element. The top of the locking collar extends upward, beyond the top of the lower section, and is disposed above the lower lip of the receiving channel. The rod of the implantation apparatus is then provided into the side receiving locus, and is positioned so that it rests snugly within the opposing grooves of the top of the locking collar. Once the rod has been properly positioned, the securing sleeve is placed onto the coupling element, with the top of the rod resting in the opposing grooves thereof. The top locking nut is then introduced onto the top of the coupling element.

The final act of driving the top locking nut down onto the upper portion of the coupling element causes the rod securing sleeve to fully descend, therein translating the rod and the locking collar therebelow downward, locking the rod between the two pair of grooves of the sleeve and the locking collar, respectively, and causing the locking collar to secure the angulation of the coupling element to the head of the screw.

It shall be understood that the securing sleeve may extend downward far enough to engage the locking collar as well, adding to the force which causes the collar to translate down and crush lock the head of the screw within the interior volume.

In a second embodiment of the pedicle screw, the rod is received from the top of the coupling element. The coupling element may be conceptually divided into a lower socket portion, and a top rod receiving portion. The socket portion is the same as in the first embodiment. The top rod receiving portion of the coupling element comprises a central channel formed vertically downward into the body of the coupling element. More particularly, from a position above the lower portion, a section of the generally cylindrical body which extends upward therefrom is removed therein providing a receiving locus into which a support rod may nest. The top portion of the coupling element, therefore, comprises a U-shape, the inner surfaces of the top portion being spaced apart sufficiently to receive the support rod therein. In other words, the upper portion comprises a pair of upwardly extending members, spaced laterall from one another, between which members is a U-shaped channel.

In this embodiment, in order that the rod may be securely held within the receiving locus, an external rod securing sleeve as above may be provided. In the alternative, the top locking nut may be sufficient. The exterior surface of the uppermost section of the top rod receiving portion of the coupling element comprises a threading onto which a locking nut may be inserted, therein locking the rod and/or the securing sleeve onto the coupling element. The bottom surface of the nut is designed to mate with either the top edge of the rod securing element or directly to the rod. It is the engagement of the nut with the upper portion of the coupling element, and the driving of the nut downward onto the upper portion of the securing sleeve or the rod which causes the rod to be locked in position. The rod is, therefore, locked between the curvate bottom of the U-shaped rod receiving locus, and the curvate top of the U-shaped rod securing sleeve or the nut itself

As stated above, with respect to the first embodiment, the bottom edge of the rod securing sleeve and/or the rod itself are designed to mate with the upper surface of the locking collar. When the nut is driven downward, therein driving the rod securing sleeve and/or rod downward as well, the locking collar descends as described above with respect to the side loading embodiment, locking the screw within the curvate interior volume.

In alternate embodiments, the polyaxial colletted taper locking mechanism may be used in side or top loading lamina hook assembly. The assembly comprises a curvate flat blade portion which has a ball shaped head. The corresponding coupling element, of the side or top loading embodiments set forth above, is mounted on the ball shaped (semi-spherical) head so that it is rotationally free prior to secure fixation of the rod thereto, and which is securely locked in a given angulation once the rod is received by the coupling element.

Subsequent to proper positioning of the blade portion of the hook under the corresponding lamina, the coupling of the rod to the coupling element (as set forth in more detail hereinabove), and the setting of the proper angulation of the coupling element relative to the hook, the locking collar is forced by a sufficient application of pressure downward along the exterior of the lower portion of the coupling element. The locking collar therein applies an inward force against the walls of the interior chamber, and the corresponding narrowing of the vertical slots thereof. Once fully driven downward the locking collar causes the coupling element to be securely locked relative to the blade portion of the hook.

An alternate implant device which may utilize the polyaxial colletted locking coupling element mechanism of the present invention is a sacral block. This embodiment of a sacral block includes a flat plate-like first element, which may be affixed to the sacrum by a pair of bone screws. This first element includes a ball head element disposed above the plate surface, onto which the corresponding coupling element (side or top loading) may be mounted.

In each of the embodiments described above, the inner surface of the locking collar and the outer surface of the lower socket portion of the coupling element may alternatively comprise mateable threadings, oriented such that rotation of the locking collar relative to the coupling element causes the collar to translate down the lower portion toward the bottom of the element. In these embodiment, therefore, the locking collar may be independently driven downward along the lower socket portion of the coupling element to lock the curvate head of the first element in the interior volume of the second.

### BRIEF DESCRIPTION OF THE FIGURES

Figures 1a and 1b are, respectively, side views of the side and top loading coupling elements of the present invention;
Figure 2 is a side view of the locking collar of the present invention, shown along a direction wherein the rod seating grooves thereof are aligned perpendicular to the plane of view;
Figure 3 is a side view of the top locking nut which is an aspect of the present invention;
Figure 4 is a side view of a rod securing sleeve which is utilized in embodiments of the present invention;
Figure 5 is a side view of the pedicle screw which is an aspect of certain embodiments of the present invention;
Figure 6 is a side view of the blade portion of the lamina hook aspect of the present invention;
Figure 7 is a side view of the plate portion of the sacral block aspect of the present invention;
Figures 8a and 8b are, respectively, side views of the coupling elements of Figures 1a and 1b, mounted on ball head of the type illustrated Figures 5, 6, and 7 including the locking collar of Figure 2, the locking nut of Figure 3, the rod securing sleeve Figure 4, and a rod;
Figures 9a and 9b are, respectively, side views of side and top loading coupling elements having a threading on the exterior surface of the lower portions thereof; and
Figure 10 is a side view of the threaded locking collar which is an aspect of the present invention.

### DETAILED DESCRIPTION OF THE PREFERRED EMBODIMENT

While the present invention will be described more fully hereinafter with reference to the accompanying drawings, in which particular embodiments and methods of implantation are shown, it is to be understood at the outset that persons skilled in the art may modify the invention herein described while achieving the functions and results of this invention. Accordingly, the descriptions which follow are to be understood as illustrative and exemplary of specific structures, aspects and features within the broad scope of the present invention and not as limiting of such broad scope.

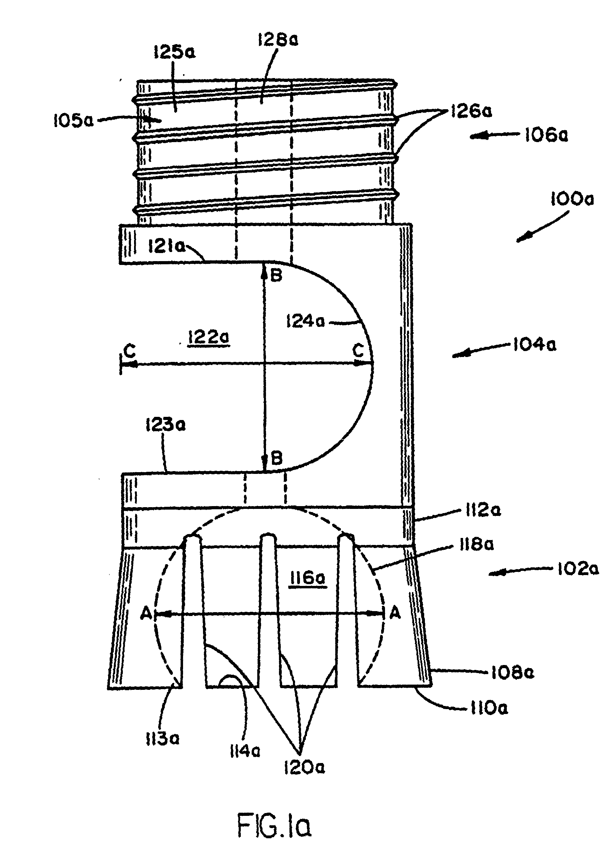

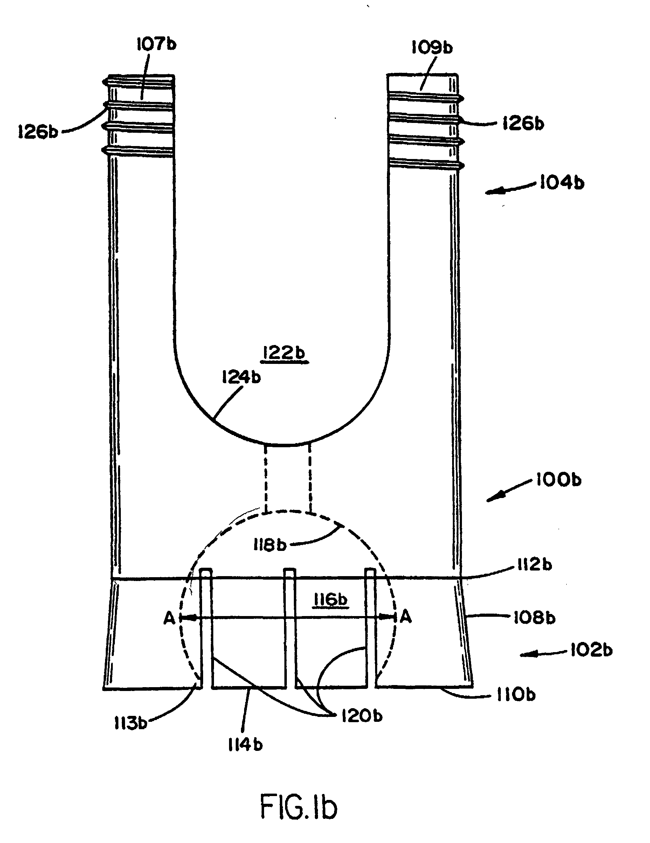

Referring now to Figures 1a and 1b, alternative preferred embodiments of the coupling element 100a,100b of the present invention is shown in side views, wherein critical features of the interior of the element are shown in phantom. The coupling elements 100a,100b each comprise a generally cylindrical body which may be conceptually separated into a lower portion 102a,102b and an upper portion 106a,106b, each of which shall be described more fully hereinbelow. The upper portion 106a of the coupling element 100a shown in Figure 1a, may be further subdivided into an intermediate portion 104a and a top portion 105a.

First, with respect to the lower portions of each element 102a,102b, which are identical, the exterior surface 108a,108b of the body is tapered in the elongate direction such that the body is wider at the bottom 110a,110b of the lower portion 102a,102b than at the top 112a,112b thereof. The bottom 110a,110b of the element 100a,100b includes an opening 114a,114b, defined by annular lip 113a,113b, which forms the mouth of an interior chamber 116a,116b. The diameter of the opening 114a,114b, when otherwise unaffected by external deflecting forces, is more narrow than the maximum diameter A-A of the interior chamber 116a,116b. The interior chamber 116a,116b has a generally curvate inner surface 118a,118b which is correspondingly shaped to receive a semi-spherical shaped object.

The exterior surface of the lower portion 102a,102b includes a series of slots 120a,120b which extend vertically upward from the bottom 110a,110b of the element 100a,100b to a point which is closer to the top 112a, 112b of the lower portion 102a,102b than the maximum horizontal diameter A-A. The slots 120a,120b are provided in order that the application of an external deflecting force may widen or narrow the opening 114a, 114b therein permitting the insertion of an object which is larger than the undeflected diameter of the opening 114a,114b, or conversely, providing for the retention of an object which is smaller than the undeflected diameter of the opening 114a, 114b.

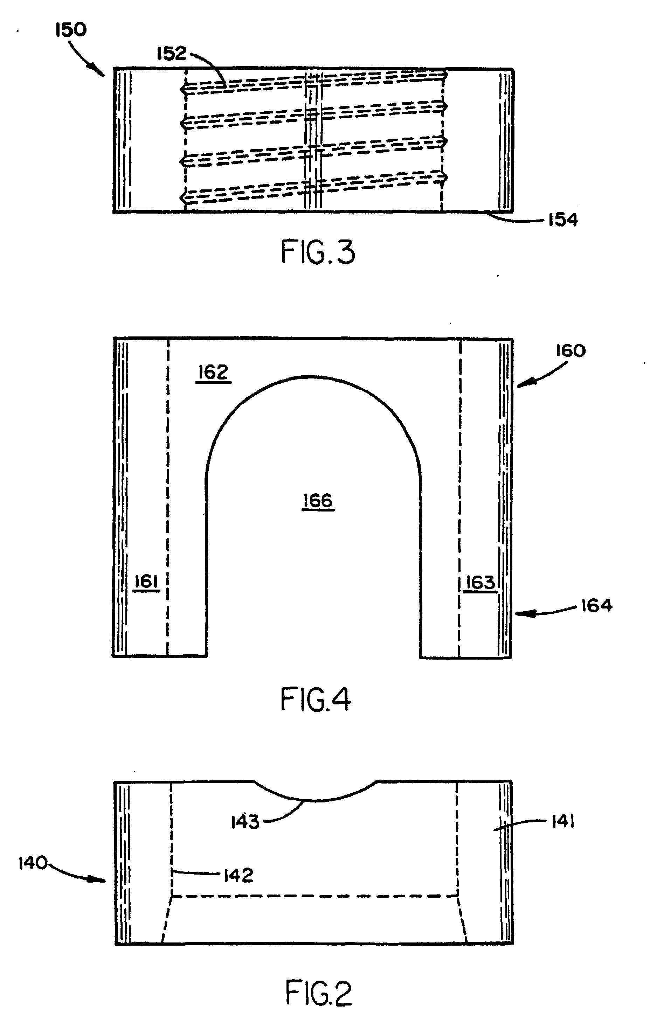

With specific reference to Figure 1a, the intermediate portion 104a of the generally cylindrical body of the coupling element 100a includes a large horizontal channel 122a, a rod receiving locus, in the side of the coupling element 100a. The channel 122a comprises a curvate inner wall 124a. In the embodiment shown in Figure 1a, the vertical distance B-B from the top 121a of the channel 122a to the bottom 123a thereof, is larger than the diameter of the rod which is to be provided therein. This distance B-B is necessarily larger than the diameter of the rod so that the rod may be translated upward and downward within this channel 122a. In addition, the maximum channel vertical dimension C-C is such that the support rod which is positioned therein nests fully within the coupling element 100a, and does not extend beyond the lateral extent thereof (which would prevent a rod securing sleeve, as shall be described with reference to Figure 4 from sliding into retaining relationship with the rod within the channel 122a).

Further with respect to Figure 1a, the top portion 105a of the coupling element 100a comprises a slightly narrower cylindrical core 125a, having a threading 126a thereon. This top portion 105a, and the threading 126a thereon, is ideally suited for receiving a top locking nut (see Figure 3).

Additionally, an axial bore 128a extends through the top portion 105a, through the intermediate portion 104a, and into the lower portion 102a. The bore 128a provides a linear passage through which a user may insert a screw-driving tool to access the ball head in the interior chamber 116a, and any structural elements therein.

Referring now to Figure 1b, upper portion 104b of the generally cylindrical body of the coupling element 100b comprises a pair of upwardly extending members 107b,109b defining therebetween a vertically oriented channel 122b in the top of the coupling element 100b. The channel 122b comprises a curvate bottom surface 124b which, for example defines a semi-circular cross-section. The depth of the channel 122b is such that a support rod which is positioned therein may nests fully within the coupling element 100b, the top of the rod thereby being positioned substantially below the top of the upper portion. This permits the top locking nut (see Figure 3) to be disposed on the top of the coupling element in a manner described more fully hereinbelow.

The upper portion 104b of the coupling element 100b, which comprises a pair of spaced apart upwardly extending members 107b,109b, also comprises an external surface threading 126b. These members 107b,109b, and the threading 126b thereon, are ideally suited for receiving a top locking nut (see Figure 3).

Referring now to Figure 2, the locking collar 140 comprises a short and hollow tubular body 141 having a pair of opposing grooves 143. The grooves 143 are provided for the rod to seat against in the collar's initial disposition. The interior surface 142 of the locking collar 140 also includes a taper. The collar 140 is designed to translate downward along the lower portion 102a,102b of the coupling element 100a,100b to cause the contraction of the interior volume 116a, 116b, thereof, thereby locking therein a ball which had previously been polyaxially retained therein. The mutual tapering of the collar 140 and the lower portions 102a,102b of the coupling elements 100a,100b eliminates means by which the relative motion of the collar 140 and the coupling element 1 00a, 100b may bind before causing the crush locking of the ball in the interior volume 116a,116b

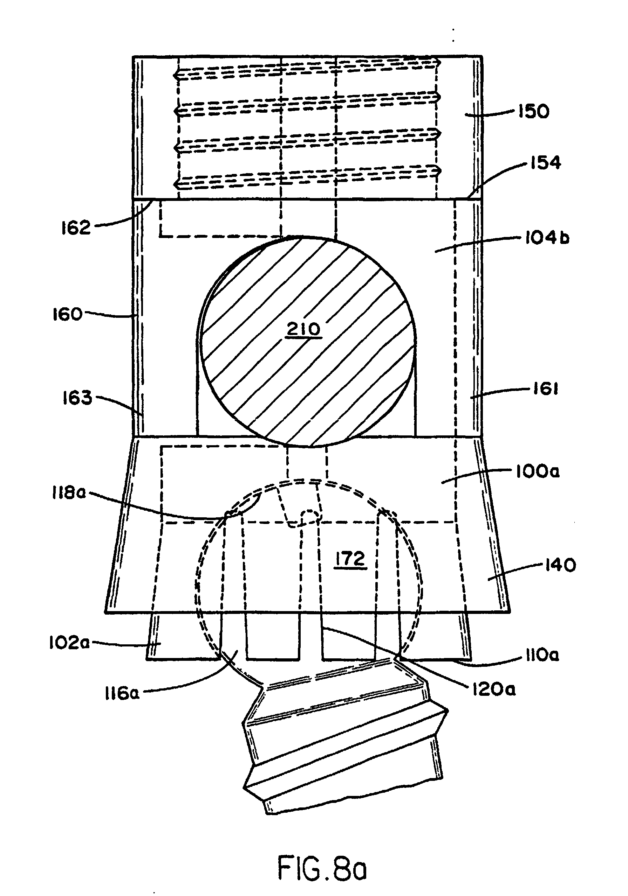

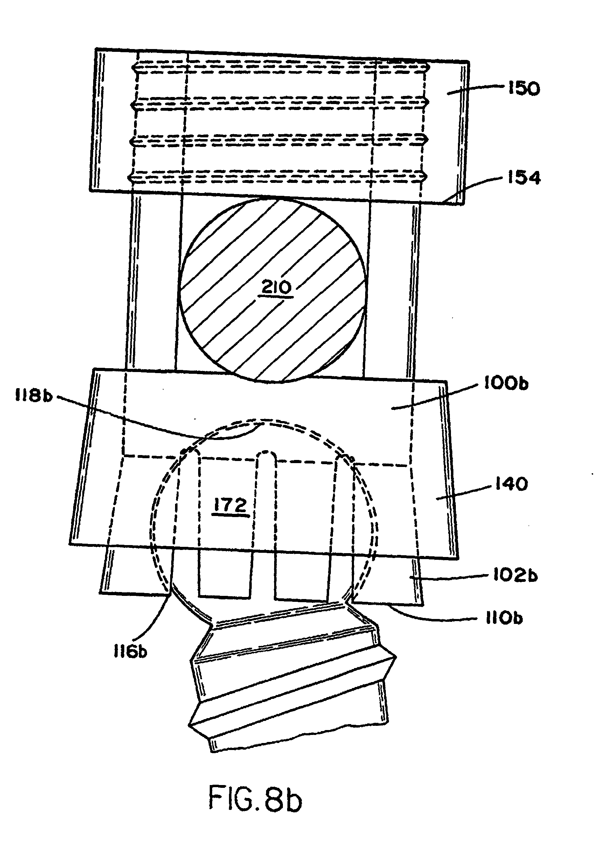

Referring specifically to Figure 3, the top locking nut 150 comprises an inner threading 152 which is intended to mate with the threading 126a,126b on the upper portions 106a,106b of the coupling elements 100a,100b. The bottom surface 154 of the nut 150 is intended to seat against either the top surface of the rod, or against the top surface of the rod securing sleeve (see Figures 4 and 8a) but is permitted to rotate relative to the sleeve, therein providing a means for driving the sleeve and/or rod downward (as more fully described hereinbelow with respect to the full assembly of the device, and with respect to Figures 8a and 8b).

Referring now specifically to Figure 4, and the rod securing sleeve 160 shown therein, the sleeve comprises a hollow cylindrical body 162 having an interior diameter which is equal to the outer diameter of the coupling element 100a, so that it may be placed thereover. The bottom portion 164 of the rod securing sleeve 160 comprises a pair of downwardly extending members 161,163 which define, therebetween, a second channel 166 through which the rod passes. The rod securing sleeve is therefore, introduced over the upper portion 106a of the coupling element 100a, once the rod has been inserted in the side channel 122a thereof. The downward translation of the rod securing sleeve 160 causes the rod to translate downwardly within the channel 122a, along with the locking collar 140, until the locking collar 140 locks the ball head in the interior volume 122a of the coupling element 102a, and the rod is locked in the channel 122a.

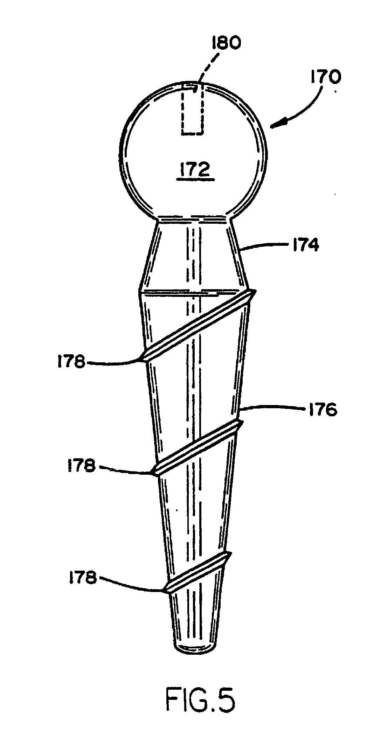

Referring now to Figure 5, a side view of the screw portion of the present invention, comprising a curvate head, is shown. The screw 170 comprises a head portion 172, a neck 174, and a shaft 176. In Figure 5, the shaft 176 is shown as having a tapered shape with a high pitch thread 178. It shall be understood that a variety of shaft designs are interchangeable with the present design. The specific choice of shaft features, such as thread pitch, shaft diameter to thread diameter ratio, and overall shaft shape, should be made be the physician with respect to the conditions of the individual patient's bone, however, this invention is compatible with a wide variety of shaft designs.

The head portion 172 of the screw 170 comprises a semi-spherical shape, which has a recess 180 in it. It is understood that the semi-spherical shape is a section of a sphere, in the embodiment shown the section is greater in extent than a hemisphere, and it correspondingly exhibits an external contour which is equidistant from a center point of the head. In a preferred embodiment, the major cross-section of the semi-spherical head 172 (as shown in the two dimensional illustration of Figure 5) includes at least 270 degrees of a circle.

The recess 180 defines a receiving locus for the application of a torque for driving the screw 170 into the bone. The specific shape of the recess 172 may be chosen to cooperate with any suitable screw-driving tool. For example, the recess 180 may comprise a slot for a flat-headed screwdriver, a crossed recess for a phillips head screwdriver, or most preferably, a hexagonally shaped hole for receiving an allen wrench. It is further preferable that the recess 180 be co-axial with the general elongate axis of the screw 170, and most particularly with respect to the shaft 176. Having the axes of the recess 180 and the shaft 176 co-linear facilitates step of inserting the screw 170 into the bone.

The semi-spherical head portion 172 is connected to the shaft 176 at a neck portion 174. While it is preferable that the diameter of the shaft 176 be less than the diameter of the semi-spherical head 172, it is also preferable that the neck 174 of the screw 170 be narrower than the widest portion of the shaft 176. This preferable dimension permits the screw to be locked at a variety of angles while still being securely joined to the coupling element.

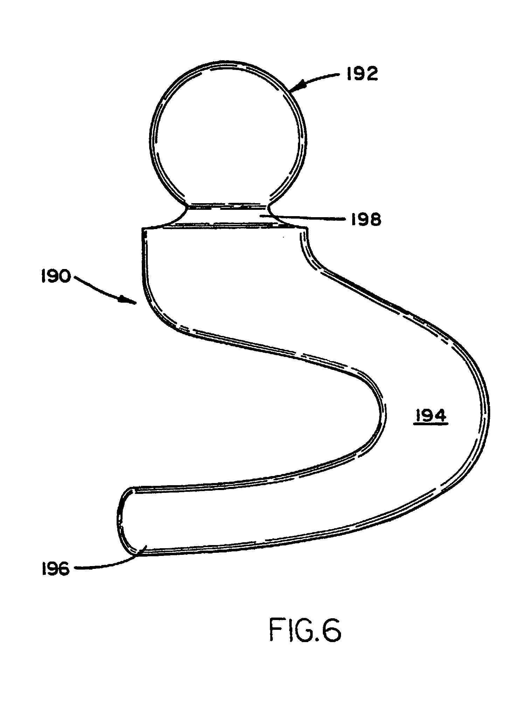

Referring now to Figure 6, a side view of the blade portion 190 of the hook device is provided. The blade portion 190 comprises a head portion 192 and a C-shaped portion 194. The lower extending branch 196 of the C-shaped portion 194 comprises a flat member which is understood to be the portion which is inserted under the lamina of the patient's spine. The semi-spherical head portion 192 is connected to upper extending branch of the C-shaped portion 194 at a neck portion 198.

The head portion 192 of the blade portion comprises a semi-spherical shape. It is understood that the semi-spherical shape is a section of a sphere. In the embodiment shown, the section is greater in extent than a hemisphere, and it correspondingly exhibits an external contour which is equidistant from a center point of the head. In a preferred embodiment, the major cross-section of the semi-spherical head 192 includes at least 270 degrees of a circle.

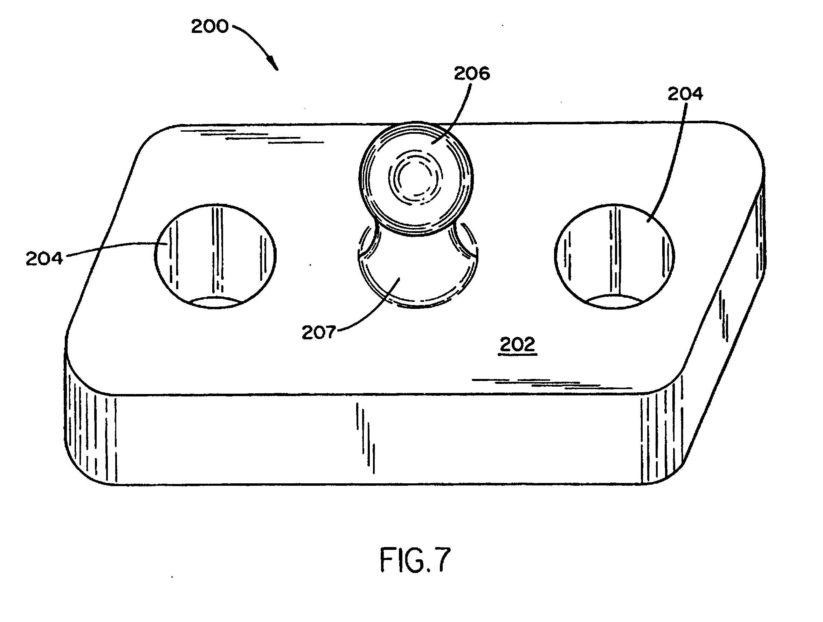

Referring now to Figure 7, a side perspective view of the sacral block 200 is provided. The block 200 comprises a generally planar portion 202 having a pair of through holes 204 therein for receiving therethrough bone screws so that it may be secured to a sacrum. It further includes an upwardly projecting semi-spherical ball 206, which is mounted on a wide short post or neck 207. This semi-spherical ball 206, as are the semi-spherical ball heads of the screw and blade 172 and 192, respectively, is provided for insertion into, and subsequent locking within, the interior volume 116a,116b of the poly axial coupling element 100a,100b.

Referring now to Figures 8a and 8b, the coupling elements 100a and 100b, as described more fully above with respect to Figures 1a and 1b, respectively, are shown in side views. In these views: (1) a semi-spherical ball head 172, 192,206 of the screw 170 or blade 190 or block 200 has been received within the interior chamber 116a, 116b; (2) the locking collar 140 is shown in its locked position about the lower portion 102a,102b; (3) the top locking nut 150 is threaded onto the upper portion 104a,104b; and (4) on the side loading coupling element 100a only, the rod securing sleeve 160 has been positioned over the coupling element 100a to retain the rod in the element.

Prior to full assembly, the head 172, 192, or 206 of corresponding elements, is free to move polyaxially relative to the coupling element 100a,100b, however, it is prevented from fully separating from the interior chamber 116a,116b by the annular lip 113a,113b at the bottom 110a,110b of the lower portion 102a,102b.

Implantation of these implant devices is preceded by the proper preparation of the implantation site. (For example, with a pedicle screw embodiment, a pre-drilled hole is provided in the bone, into which it is desired that the screw 170 may be inserted.) The head 172,192,206 is inserted into the interior chamber 116a, 116b of the coupling element 100a,100b. As stated above, at this point in the assembly process, the locking collar 140 has not yet been forced downward along the outwardly tapered lower portion 102a,102b, thereby permitting rotational and polyaxial relative motion.

Once the coupling element 100a,100b and the ball head are properly aligned, the screw, hook, or sacral block is affixed to the appropriate prepared site and the support rod 210 is nested within the channel 122a, 122b, and disposed on the grooves 143 of the locking collar 140. In the case of the side loading coupling element 100a, the rod securing sleeve 160 is then dropped over the element 100a, such that the grooves 143 of the sleeve 160 are seated against the top of the rod 210. In the top loading embodiment 100b, the rod securing sleeve 160 is not necessary.

With either embodiment, once the proper angulation of the coupling element to the head 172,192,206 and the secure nesting of the rod 210 in the channel 122a,122b on the locking collar 140 have been established, the top locking nut 150 is threaded onto the threading 126a,126b of the upper portion 106a,106b.

In the side loading embodiment 100a, the bottom surface 154 of the nut 150 seats against the top surface 162 of the rod securing sleeve 160. As the nut 150 is advanced, and descends relative to the coupling element 100a, the rod securing sleeve 160 is driven downward. This motion causes the rod 210 to translate downward therein forcing the locking collar 140 to descend as well. The locking collar 140 may be driven downwardly by either interaction solely with the rod 210, or also by direct contact with the downwardly extending members 161,163 of the rod securing sleeve 160.

In the top loading embodiment 100b, in which the rod securing sleeve 160 is not utilized, the top locking nut 150 is advanced directly into contact with the rod 210, which in turn causes the collar 140 to descend.

In either case, by descending along the tapered lower portion 102a,102b of the element, the locking collar 140 provides an inwardly directed deflecting force which causes the slots 120a,120b in the lower portion 102a,102b of the element to narrow so that the collar may proceed downward. This deflection inward causes the inner surface 118a, 118b of the interior chamber 116a, 116b to crush lock against the head 172,192,206. This clamping force locks the angulation of the screw, hook, or block 170,190,200 to the coupling element 100a,100b.

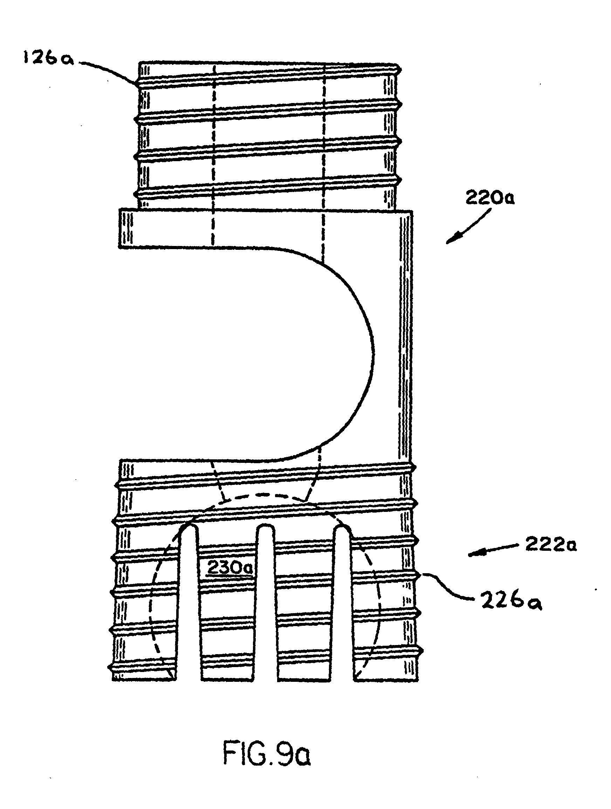

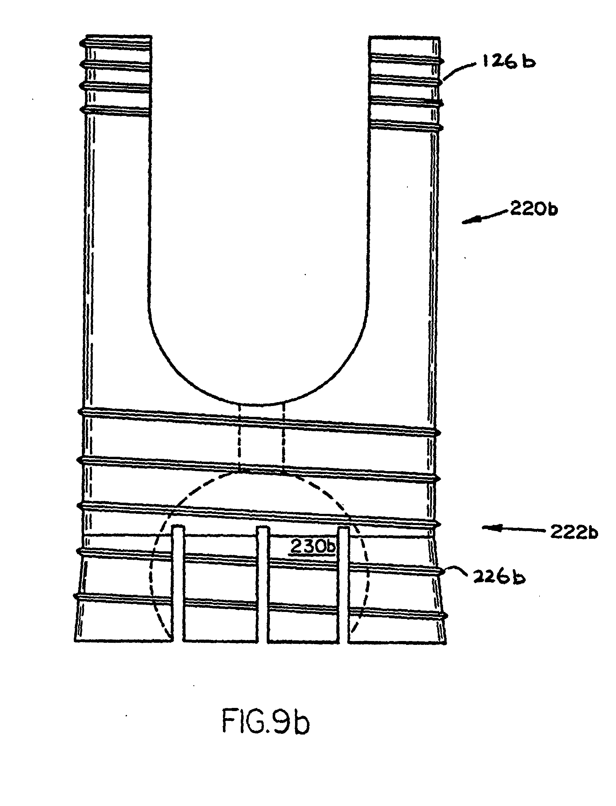

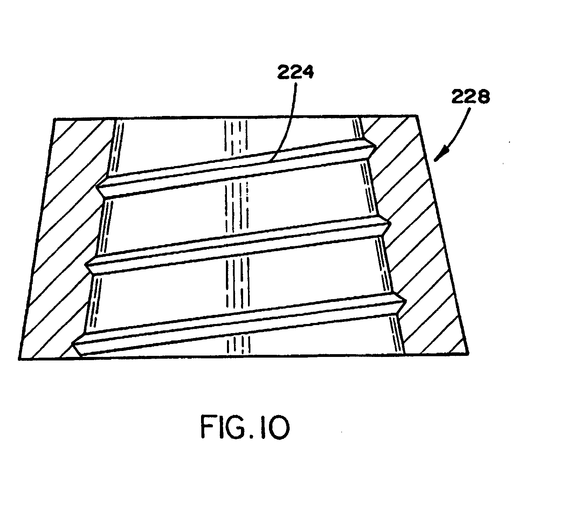

Referring now to Figures 9a and 9b, in which two alternative coupling element embodiments of the present invention are shown in side views, a threaded version of the locking mechanism is described. The coupling elements 220a,220b, being side and top loading variations, respectively, are identical to the coupling elements 100a,100b as set forth hereinabove with reference to Figures 1a and 1b, but for the threading 226a,226b disposed on the tapered lower portions 222a,222b thereof. This threading 226a,226b is provided to engage a threading 224 on a locking collar 228, as is illustrated in Figure 10. This locking collar 228 is identical to the collar 140 set forth hereinabove with reference to Figure 2, but for the interior threading 224 thereof. It shall be understood that in such an embodiment, the collar 228 is selectively advanceable to lock the head 172,192,206 in the interior volume 230a,230b of the coupling element 220a,220b independent of rod and/or securing sleeve contact therewith.

While there has been described and illustrated various embodiments of a polyaxial locking mechanism for use with posterior spinal rod implantation apparatus, it will be apparent to those skilled in the art that variations and modifications are possible without deviating from the broad spirit and principle of the present invention. The present invention shall, therefore, be limited solely by the scope of the claims appended hereto.

## Claims

1. A polyaxial locking mechanism for use with orthopaedic implantation apparatus, including, a first element (170) having a head (172), a second element (100), a portion of said second element (100) having an interior volume (116) for receiving therein said head (172), and a locking collar (140),
said head (172) and said interior volume (116) being curvate, **characterised in that** said portion of said second element (100) has a tapered and colletted exterior surface (108), and said locking collar (140) is mounted around said tapered colletted portion (108), selected translation of said locking collar (140) relative to said portion (108) causing said curvate interior volume (116) to crush lock to said curvate head (172).

2. The mechanism according to claim 1, **characterized in that** said second element (100) further comprises a rod receiving channel (122) formed therein.

3. The mechanism according to claim 2, **characterized in that** said rod receiving channel (122) is formed in the side of said second element (100).

4. The mechanism according to claim 2, **characterised in that** said rod receiving channel (122) is formed in the top of said second element (100).

5. The mechanism according to any one of claims 2-4, further comprising means for locking a rod in said rod receiving channel (122).

6. The mechanism according to claim 5, **characterized in that** said locking of said rod in said rod receiving channel (122) causes the selected translation of said locking collar (140).

7. The mechanism according to any one of claims 1-6, **characterized in that** said locking collar (140) and said tapered and colletted portion (108) each further comprise a threading (224,226), such that said selected translation of said locking collar (140) is provided by mutual engagement thereof.

8. The mechanism according to any one of claims 1-7, **characterized in that** the second element (100) further includes a surface threading (126) disposed on an upper portion (106) of the second element.

9. The mechanism according to any one of claims 1-8, **characterized in that** the interior volume (116) forms an expandable and contractable interior chamber, the interior chamber having an expandable and contractable opening (114) for receiving therethrough said curvate head (172).

10. The mechanism according to claim 9, **characterized in that** downward translation of said collar (140) causes said interior chamber (116) and said opening (114) to contract, therein locking the first element to the second element.

11. The mechanism according to claims 9 or 10, **characterized in that** said second element further comprises at least one vertical slot (120) extending upward from said opening (114), therein rendering said interior chamber (116) and said opening (114) expandable and contractable.

12. The mechanism according to any one of claims 9-11, **characterized in that** said portion of said second element (100) is wider at said opening.

13. The mechanism according to claim 12, **characterized in that** downward translation of said looking collar (140) causes the interior chamber (116) and said opening to contract.

14. The mechanism according to any one of claims 11-13, **characterized in that** said second element (100) comprises a second threading (226) disposed about an outer surface thereof, at least a portion of which includes the at least one vertical slot (120), and wherein said locking collar (140) includes a threading (224) on an interior surface thereof, mateable with said second threading (226) whereby said looking collar (140) may be downwardly translated on said second threading.

15. The mechanism according to any one of claims 1-8, **characterized in that** the second element includes a lower portion (102), having a top (112) and a bottom (110), said lower portion comprising:
a taper wherein the bottom (110) of the lower portion is wider than the top (112);
at least one vertical slot (120) extending upward from a bottom of said lower portion; and
an opening (114) in said bottom (110) of said lower portion for receiving therethrough said curvate head (172), said opening being expandable and contractable by forces applied to said at least one vertical slot (120).

16. The mechanism according to any one of claims 2-15, further comprising a rod securing sleeve (160) having opposing vertical slots (166), said sleeve being positionable about a section of said second element (100) for securing a rod within said channel.

17. The mechanism according to claims 1-16, further comprising a top locking nut (150) which is mateable with a threading (126).

18. The mechanism according to claim 17, **characterized in that** a bottom surface (154) of said top locking nut (150) seats against a top surface of said rod securing sleeve (160), the downward translation of said top locking nut (150) causing said rod securing sleeve (160) to crush lock said rod to said second element (100).

19. The mechanism according to claim 18, **characterized in that** a bottom surface (164) of said rod securing sleeve (160) seats against a top surface of said locking collar (140), whereby the downward translation of said nut (150) on said threading (226) of said second element causes the downward translation of said locking collar to crush lock the curvate head (172) within said interior chamber.

20. The mechanism according to any one of claims 1-19, **characterized in that** said first element is selected from the group consisting of a bone screw, a lamina hook, and a sacral block.

21. The mechanism according to any one of claims 1-20, **characterized in that** said curvate head is semispherical.

22. The mechanism according to claim 9, **characterised in that** translation of said collar (140) relative to said second element (100) causing said interior chamber (116) and said opening (114) to contract, therein locking the first element (170) to the second element.

23. The mechanism of any preceding claim, **characterised in that** the second element (100a) includes an axial bore (128 a) having a length that extends through an intermediate portion (104 a) and into a lower portion (102 a) of the second element (100a).

24. The mechanism according to claim 23, **characterised in that** the bore (128 a) has a diameter.

25. The mechanism according to claim 23 or claim 24 **characterised in that** the diameter varies along the length of the bore (128 a).

26. The mechanism according to any of claims 23 to 25 **characterised in that** at least one portion of the bore (128 a) tapers from a first diameter to a second diameter, and the second diameter is larger than the first diameter.

27. The mechanism according to claim 26, **characterised in that** the first diameter is located within an interior portion of the bore (128 a).

28. The mechanism according to claim 27, **characterised in that** the second diameter is located adjacent one end of the bore (128 a).

29. The mechanism according to claim 27, **characterised in that** the curvate head (172) has a top surface (118 b), and the first diameter of the bore (128 a) is located below the top surface of the curvate head, when the curvate interior volume (116) is crush locked to the curvate head (172).

30. The mechanism of any preceding claim characterised int hat the locking collar (140) has a bore (128a) and a vertically orientated channel (122b) transverse to the bore and extending from a first end of the locking collar to an interior thereof, the channel defining a curvate bottom surface (124b) and having fixed dimensions, the fixed dimensions of the channel remaining substantially unchanged when the curvate interior volume ( 116) is locked to the curvate head ( 172).

31. The mechanism according to claim 30 **characterised in that** a portion of the curvate bottom surface (124b) is U-shaped.

32. The mechanism according to claim 23, **characterised in that** the bore is configured and dimensioned to allow a user to insert a screw-driving tool to access the head (172), when the head is received in the interior chamber (116 a).

33. The mechanism according to any of claims 22 - 32, **characterised in that** said first element (170) has first and second ends and an outer surface with at least one thread (178) extending from one end of the first element to an interior thereof.

## Patentansprüche

1. Polyaxialer Verriegelungsmechanismus zur Verwendung mit orthopädischen Implantationsvorrichtungen, mit einem ersten Element (170) mit einem Kopf (172), einem zweiten Element (100), wobei ein Bereich des zweiten Elements (100) ein Innenvolumen (116) zum Aufnehmen des Kopfes (172) aufweist, und einem Verriegelungskragen (140), wobei der Kopf (172) und das Innenvolumen (116) gebogen sind, **dadurch gekennzeichnet, daß** der Bereich des zweiten Elements (100) eine zulaufende und als Spannring ausgebildete Außenfläche (108) aufweist, und der Verriegelungskragen (140) um den zulaufenden Spannbereich (108) angebracht ist, wobei eine gewählte Verschiebung des Verriegelungskragens (140) in bezug auf den Bereich (108) eine Quetschverriegelung des gebogenen Innenvolumens (116) mit dem gebogenen Kopf (172) bewirkt.

2. Mechanismus nach Anspruch 1, **dadurch gekennzeichnet, daß** das zweite Element (100) ferner einen darin ausgebildeten Stangenaufnahmekanal (122) aufweist.

3. Mechanismus nach Anspruch 2, **dadurch gekennzeichnet, daß** der Stangenaufnahmekanal (122) in der Seite des zweiten Elements (100) ausgebildet ist.

4. Mechanismus nach Anspruch 2, **dadurch gekennzeichnet, daß** der Stangenaufnahmekanal (122) in der Oberseite des zweiten Elements (100) ausgebildet ist.

5. Mechanismus nach einem der Ansprüche 2-4, ferner mit einer Einrichtung zum Verriegeln einer Stange im Stangenaufnahmekanal (122).

6. Mechanismus nach Anspruch 5, **dadurch gekennzeichnet, daß** das Verriegeln der Stange in dem Stangenaufnahmekanal (122) die gewählte Verschiebung des Verriegelungskragens (140) bewirkt.

7. Mechanismus nach einem der Ansprüche 1-6, **dadurch gekennzeichnet, daß** der Verriegelungskragen (140) und der zulaufende und als Spannring ausgebildete Bereich (108) jeweils ferner ein Gewinde (224, 226) aufweisen, so daß die gewählte Verschiebung des Verriegelungskragens (140) durch gegenseitigen Eingriff derselben bewirkt wird.

8. Mechanismus nach einem der Ansprüche 1-7, **dadurch gekennzeichnet, daß** das zweite Element (100) ferner ein Oberflächengewinde (126) aufweist, das in einem oberen Bereich (106) des zweiten Elements vorgesehen ist.

9. Mechanismus nach einem der Ansprüche 1-8, **dadurch gekennzeichnet, daß** das Innenvolumen (116) eine dehn- und zusammenziehbare innere Kammer bildet, wobei die innere Kammer eine dehn- und zusammenziehbare Öffnung (114) zum Aufnehmen des gebogenen Kopfs (172) durch diese hindurch aufweist.

10. Mechanismus nach Anspruch 9, **dadurch gekennzeichnet, daß** eine abwärts gerichtete Verschiebung des Kragens (140) ein Kontrahieren der inneren Kammer (116) und der Öffnung (114) bewirkt, wodurch das erste Element mit dem zweiten Element verriegelt wird.

11. Mechanismus nach Anspruch 9 oder 10, **dadurch gekennzeichnet, daß** das zweite Element ferner wenigstens einen vertikalen Schlitz (120) aufweist, der sich von der Öffnung (114) nach oben erstreckt, wodurch die innere Kammer (116) und die Öffnung (114) dehn- und zusammenziehbar sind.

12. Mechanismus nach einem der Ansprüche 9-11, **dadurch gekennzeichnet, daß** der Bereich des zweiten Elements (100) an der Öffnung breiter ist.

13. Mechanismus nach Anspruch 12, **dadurch gekennzeichnet, daß** die abwärts gerichtete Verschiebung des Verriegelungskragens (140) das Zusammenziehen der inneren Kammer (116) und der Öffnung bewirkt.

14. Mechanismus nach einem der Ansprüche 11-13, **dadurch gekennzeichnet, daß** das zweite Element (100) ein zweites Gewinde (226) um eine Außenfläche desselben aufweist, von der zumindest ein Bereich den wenigstens einen vertikalen Schlitz (120) enthält, und wobei der Verriegelungskragen (140) ein Gewinde (224) auf einer Innenfläche aufweist, das in Eingriff mit dem zweiten Gewinde (226) bringbar ist, wodurch der Verriegelungskragen (140) auf dem zweiten Gewinde nach unten verschiebbar ist.

15. Mechanismus nach einem der Ansprüche 1-8, **dadurch gekennzeichnet, daß** das zweite Element einen unteren Bereich (102) mit einer Oberseite (112) und einer Unterseite (110) hat, wobei der untere Bereich aufweist:
eine Verjüngung, wobei die Unterseite (110) des unteren Bereichs breiter als die Oberseite (112) ist;
wenigstens einen vertikalen Schlitz (120), der sich von der Unterseite des unteren Bereichs aus aufwärts erstreckt; und
eine Öffnung (114) in der Unterseite (110) des unteren Bereichs zum Aufnehmen des gebogenen Kopfes (172) durch diese hindurch, wobei die Öffnung durch auf den wenigstens einen vertikalen Schlitz (120) aufgebrachte Kräfte dehn- und zusammenziehbar ist.

16. Mechanismus nach einem der Ansprüche 2-15, ferner mit einer Stangenbefestigungshülse (160) mit gegenüberliegenden vertikalen Schlitzen (166), wobei die Hülse um einen Abschnitt des zweiten Elements (100) positionierbar ist, um eine Stange in dem Kanal zu befestigen.

17. Mechanismus nach einem der Ansprüche 1-16, ferner mit einer oberen Verriegelungsmutter (150), die mit einem Gewinde (126) in Eingriff bringbar ist.

18. Mechanismus nach Anspruch 17, **dadurch gekennzeichnet, daß** die Unterseite (154) der oberen Verriegelungsmutter (150) auf der Oberseite der Stangenbefestigungshülse (160) sitzt, wobei die abwärts gerichtete Verschiebung der oberen Verriegelungsmutter (150) bewirkt, daß die Stangenbefestigungshülse (160) die Stange quetschend mit dem zweiten Element (100) verriegelt.

19. Mechanismus nach Anspruch 18, **dadurch gekennzeichnet, daß** die Unterseite (164) der Stangenbefestigungshülse (160) auf der Oberseite des Verriegelungskragens (140) sitzt, wodurch die abwärts gerichtete Verschiebung der Mutter (150) auf dem Gewinde (126) des zweiten Elements die abwärts gerichtete Verschiebung des Verriegelungskragens bewirkt, um den gebogenen Kopf (172) in der inneren Kammer quetschend zu verriegeln.

20. Mechanismus nach einem der Ansprüche 1-19, **dadurch gekennzeichnet, daß** das erste Element aus der Gruppe gewählt ist, die aus einer Knochenschraube, einem Lamina-Haken und einem Kreuzbeinblock besteht.

21. Mechanismus nach einem der Ansprüche 1-20, **dadurch gekennzeichnet, daß** der gebogene Kopf halbkugelförmig ist.

22. Mechanismus nach Anspruch 9, **dadurch gekennzeichnet, daß** die Verschiebung des Kragens (140) in bezug auf das zweite Element (100) das Zusammenziehen der inneren Kammer (116) und der Öffnung (114) bewirkt, wodurch das erste Element (170) mit dem zweiten Element verriegelt wird.

23. Mechanismus nach einem der vorhergehenden Ansprüche, **dadurch gekennzeichnet, daß** das zweite Element (100a) eine Axialbohrung (128a) mit einer Länge aufweist, die sich durch einen Mittelbereich (104a) und in einen unteren Bereich (102a) des zweiten Elements (100a) erstreckt.

24. Mechanismus nach Anspruch 23, **dadurch gekennzeichnet, daß** die Bohrung (128a) einen Durchmesser aufweist.

25. Mechanismus nach Anspruch 23 oder Anspruch 24, **dadurch gekennzeichnet, daß** der Durchmesser über die Länge der Bohrung (128a) variiert.

26. Mechanismus nach einem der Ansprüche 23 bis 25 **dadurch gekennzeichnet, daß** wenigstens ein Teil der Bohrung (128a) konisch von einem ersten Durchmesser zu einem zweiten Durchmesser verläuft, und der zweite Durchmesser größer als der erste Durchmesser ist.

27. Mechanismus nach Anspruch 26, **dadurch gekennzeichnet, daß** der erste Durchmesser sich in einem Innenbereich der Bohrung (128a) befindet.

28. Mechanismus nach Anspruch 27, **dadurch gekennzeichnet, daß** der zweite Durchmesser nahe einem Ende der Bohrung (128a) angeordnet ist.

29. Mechanismus nach Anspruch 27, **dadurch gekennzeichnet, daß** der gebogene Kopf (172) eine Oberseite (118b) aufweist, und der erste Durchmesser der Bohrung (128a) unter der Oberseite des gebogenen Kopfs angeordnet ist, wenn das gebogene innere Volumen (116) mit dem gebogenen Kopf (172) durch Quetschen verriegelt ist.

30. Mechanismus nach einem der vorhergehenden Ansprüche, **dadurch gekennzeichnet, daß** der Verriegelungsriegel (140) eine Bohrung (128a) und einen vertikal ausgerichteten Kanal (122b) aufweist, der quer zur Bohrung verläuft und sich von einem ersten Ende des Verriegelungskragens zur Innenseite desselben erstreckt, wobei der Kanal eine gebogene Unterseite (124b) bildet und feste Abmessungen aufweist, die im wesentlichen unverändert bleiben, wenn das gebogene innere Volumen (116) mit dem gebogenen Kopf (172) verriegelt ist.

31. Mechanismus nach Anspruch 30, **dadurch gekennzeichnet, daß** ein Bereich der gebogenen Fläche (124b) U-förmig ist.

32. Mechanismus nach Anspruch 23, **dadurch gekennzeichnet, daß** die Bohrung derart konfiguriert und bemessen ist, daß einem Benutzer möglich ist, ein Schraubendrehwerkzeug zum Zugriff auf den Kopf (172) einzuführen, wenn der Kopf in der inneren Kammer (116a) aufgenommen ist.

33. Mechanismus nach einem der Ansprüche 22-32, **dadurch gekennzeichnet, daß** das erste Element (170) ein erstes und ein zweites Ende und eine Außenfläche mit wenigstens einem Gewinde (178) aufweist, das sich von einem Ende des ersten Elements zu Innenseite desselben erstreckt.

## Revendications

1. Mécanisme de blocage polyaxial destiné à être utilisé avec un appareil d'implantation orthopédique, comprenant un premier élément (170) ayant une tête (172), un second élément (100), une partie dudit second élément (100) ayant un volume intérieur (116) destiné à recevoir ladite tête (172) et une bague de blocage (140),
ladite tête (172) et ledit volume intérieur (116) étant arrondis, **caractérisé en ce que** ladite partie dudit second élément (100) présente une surface extérieure conique et décolletée (108), et ladite bague de blocage (140) est montée autour de ladite surface conique décolletée (108), une translation choisie de ladite bague de blocage (140) par rapport à ladite partie (108) amenant ledit volume intérieur arrondi (116) à bloquer par écrasement ladite tête arrondie (172).

2. Mécanisme selon la revendication 1, **caractérisé en ce que** ledit second élément (100) présente en outre une rainure (122) de réception d'une tige formée dans cet élément.

3. Mécanisme selon la revendication 2, **caractérisé en ce que** ladite rainure (122) de réception de tige est formée dans le côté dudit second élément (100).

4. Mécanisme selon la revendication 2, **caractérisé en ce que** ladite rainure (122) de réception de tige est formée dans le dessus dudit second élément (100).

5. Mécanisme selon l'une quelconque des revendications 2 à 4, comportant en outre un moyen pour bloquer une tige dans ladite rainure (122) de réception de tige.

6. Mécanisme selon la revendication 5, **caractérisé en ce que** ledit blocage de ladite tige dans ladite rainure (122) de réception de tige provoque la translation choisie de ladite bague de blocage (140).

7. Mécanisme selon l'une quelconque des revendications 1 à 6, **caractérisé en ce que** ladite bague de blocage (140) et ladite partie conique et décolletée (108) comportent en outre chacune un filetage (224, 226), de façon que ladite translation choisie de ladite bague de blocage (140) soit produite par leur engagement mutuel.

8. Mécanisme selon l'une quelconque des revendications 1 à 7, **caractérisé en ce que** le second élément (100) comporte en outre un filetage de surface (126) disposé sur une partie supérieure (106) du second élément.

9. Mécanisme selon l'une quelconque des revendications 1 à 8, **caractérisé en ce que** le volume intérieur (116) forme une chambre intérieure pouvant se dilater et se contracter, la chambre intérieure ayant une ouverture (114) pouvant être dilatée et contractée pour recevoir à travers elle ladite tête arrondie (172).

10. Mécanisme selon la revendication 9, **caractérisé en ce qu'**une translation vers le bas de ladite bague (140) provoque une contraction de ladite chambre intérieure (116) et de ladite ouverture (114), bloquant ainsi le premier élément au second élément.

11. Mécanisme selon les revendications 9 ou 10, **caractérisé en ce que** ledit second élément présente en outre au moins une fente verticale (120) s'élevant depuis ladite ouverture (114), permettant ainsi à ladite chambre intérieure (116) et à ladite ouverture (114) d'être dilatées et contractées.

12. Mécanisme selon l'une quelconque des revendications 9 à 11, **caractérisé en ce que** ladite partie dudit second élément (100) est plus large à ladite ouverture.

13. Mécanisme selon la revendication 12, **caractérisé en ce qu'**une translation vers le bas de ladite bague de blocage (140) provoque une contraction de la chambre intérieure (116) et de ladite ouverture.

14. Mécanisme selon l'une quelconque des revendications 11 à 13, **caractérisé en ce que** ledit second élément (100) comporte un second filetage (226) disposé autour d'une surface extérieure de cet élément, dont au moins une partie comprend la, au moins une, fente verticale (120), et dans lequel ladite bague de blocage (140) comporte un filetage (224) sur une surface intérieure de cette bague, pouvant être accouplé avec ledit second filetage (226), grâce à quoi ladite bague de blocage (140) peut être translatée vers le bas sur ledit second filetage.

15. Mécanisme selon l'une quelconque des revendications 1 à 8, **caractérisé en ce que** le second élément comprend une partie inférieure (102), ayant un haut (112) et un bas (110), ladite partie inférieure comportant :
une conicité selon laquelle le bas (110) de la partie inférieure est plus large que le haut (112) ;
au moins une fente verticale (120) s'élevant depuis le bas de ladite partie inférieure ; et
une ouverture (114) dans ledit bas (110) de ladite partie inférieure destinée à recevoir à travers elle ladite tête arrondie (172), ladite ouverture pouvant être dilatée et contractée par des forces appliquées à ladite, au moins une, fente verticale (120).

16. Mécanisme selon l'une quelconque des revendications 2 à 15, comportant en outre un manchon (160) de fixation de tige ayant des fentes verticales opposées (166), ledit manchon pouvant être positionné autour d'une section dudit second élément (100) pour fixer une tige dans ladite rainure.

17. Mécanisme selon les revendications 1 à 16, comportant en outre un écrou supérieur (150) de blocage qui peut être accouplé avec un filetage (126).

18. Mécanisme selon la revendication 17, **caractérisé en ce qu'**une surface inférieure (154) dudit écrou supérieur (150) de blocage porte contre une surface supérieure dudit manchon (160) de fixation de tige, la translation vers le bas dudit écrou supérieur (150) de blocage amenant ledit manchon (160) de fixation de tige à bloquer par écrasement ladite tige audit second élément (100).

19. Mécanisme selon la revendication 18, **caractérisé en ce qu'**une surface inférieure (164) dudit manchon (160) de fixation de tige porte contre une surface supérieure de ladite bague (140) de blocage, grâce à quoi la translation vers le bas dudit écrou (150) sur ledit filetage (226) dudit second élément provoque une translation vers le bas de ladite bague de blocage pour bloquer par écrasement la tête arrondie (172) dans ladite chambre intérieure.

20. Mécanisme selon l'une quelconque des revendications 1 à 19, **caractérisé en ce que** ledit premier élément est choisi dans le groupe constitué d'une vis pour os, d'un crochet pour pédicule vertébral et un bloc pour le sacrum.

21. Mécanisme selon l'une quelconque des revendications 1 à 20, **caractérisé en ce que** ladite tête arrondie est hémisphérique.

22. Mécanisme selon la revendication 9, **caractérisé par** une translation de ladite bague (140) par rapport audit second élément (100) provoquant une contraction de ladite chambre intérieure (116) et de ladite ouverture (114), bloquant ainsi le premier élément (170) au second élément.

23. Mécanisme selon l'une quelconque des revendications précédentes, **caractérisé en ce que** le second élément (100a) présente une lumière axiale (128a) ayant une longueur qui s'étend à travers une partie intermédiaire (104a) et jusque dans une partie inférieure (102a) du second élément (100a).

24. Mécanisme selon la revendication 23, **caractérisé en ce que** la lumière (128a) a un diamètre.

25. Mécanisme selon la revendication 23 ou la revendication 24, **caractérisé en ce que** le diamètre varie sur la longueur de la lumière (128a).

26. Mécanisme selon l'une quelconque des revendications 23 à 25, **caractérisé en ce qu'**au moins une partie de la lumière (128a) est conique d'un premier diamètre à un second diamètre, et le second diamètre est plus grand que le premier diamètre.

27. Mécanisme selon la revendication 26, **caractérisé en ce que** le premier diamètre est situé dans une partie intérieure de la lumière (128a).

28. Mécanisme selon la revendication 27, **caractérisé en ce que** le second diamètre est situé de façon à être adjacent à une extrémité de la lumière (128a).

29. Mécanisme selon la revendication 27, **caractérisé en ce que** la tête arrondie (172) présente une surface supérieure (118b), et le premier diamètre de la lumière (128a) est situé en dessous de la surface supérieure de la tête arrondie, lorsque le volume intérieur arrondi (116) est bloqué par écrasement sur la tête arrondie (172).

30. Mécanisme selon l'une quelconque des revendications précédentes, **caractérisé en ce que** la bague (140) de blocage présente une lumière (128a) et un canal (122b) orienté verticalement, transversal à la lumière et s'étendant depuis une première extrémité de la bague de blocage vers l'intérieur de celle-ci, le canal définissant une surface inférieure arrondie (124b) et ayant des dimensions fixes, les dimensions fixes du canal restant sensiblement inchangées lorsque le volume intérieur arrondi (116) est bloqué sur la tête arrondie (172).

31. Mécanisme selon la revendication 30, **caractérisé en ce qu'**une partie de la surface arrondie (124b) est en forme de U.

32. Mécanisme selon la revendication 23, **caractérisé en ce que** la lumière est configurée et dimensionnée pour permettre à un utilisateur d'introduire un tournevis pour accéder à la tête (172) lorsque la tête est reçue dans la chambre intérieure (116a).

33. Mécanisme selon l'une quelconque des revendications 22 à 32, **caractérisé en ce que** ledit premier élément (170) présente des première et seconde extrémités et une surface extérieure avec au moins un filetage (178) s'étendant d'une extrémité du premier élément vers l'intérieur de celui-ci.
